(19) Europäisches Patentamt
European Patent Office
Office européen des brevets

(11) Publication number: **0 404 115 A1**

## (12) EUROPEAN PATENT APPLICATION

(21) Application number: **90111675.6**

(51) Int. Cl.5: **C07D 207/08, A61K 31/40**

(22) Date of filing: **20.06.90**

(30) Priority: **21.06.89 US 369366**
**14.05.90 US 523293**

(43) Date of publication of application:
**27.12.90 Bulletin 90/52**

(84) Designated Contracting States:
**AT BE CH DE DK ES FR GB GR IT LI LU NL SE**

(71) Applicant: **SYNTEX (U.S.A.) INC.**
**3401 Hillview Avenue**
**Palo Alto California 94304(US)**

(72) Inventor: **Fisher, Lawrence E.**
**1036 Marilyn Drive**
**Mountain View, California 94040(US)**
Inventor: **Muchowski, Joseph M.**
**1720 Banff Drive**
**Sunnyvale, California 94087(US)**
Inventor: **Galeazzi, Edvige**
**Dr. Vertiz 882**
**Col. Narvate, 03020 Mexico, D.F.(MX)**
Inventor: **Rosenkranz, Roberto P.**
**7 Brittany Meadows**
**Atherton, California 9402725(US)**
Inventor: **McClelland, Deborah L.**
**4119 Park Boulevard**
**Palo Alto, California 94306(US)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40(DE)**

(54) **Bis(benzylpyrrolidine)Derivatives as dopamine agonists.**

(57) Dopamine agonist compounds useful in the treatment of hypertension and congestive heart failure in mammals are disclosed. The dopamine agonist compounds include racemic and non-racemic mixtures of chiral compounds as well as optically pure R,R; R,S and S,S stereoisomers which are all intended to be schematically described by the following general formula (I)

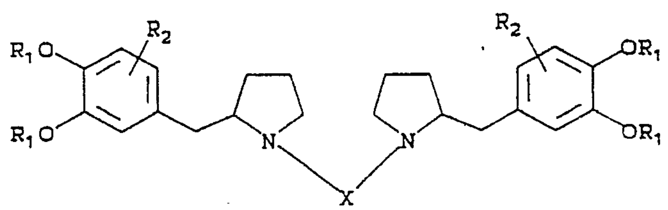

(I)

wherein:

$R_1$ is independently hydrogen, lower alkyl, $-C(O)R_3$, or $-C(O)NR_3R_4$, wherein

$R_3$ and $R_4$ are independently lower alkyl, optionally substituted phenyl or phenyl lower alkyl;

$R_2$ is hydrogen or lower alkyl-,

X is $-(CH_2)_m-$ or $-(CH_2)_nY(CH_2)_n-$, where

m is an integer from 1 to 10, n is an integer of 1 to 3, and Y is oxygen or sulfur;

All of $R_1$, and $R_2$ are most preferably hydrogen, X is most preferably $-(CH_2)_m-$ and the preferred optically pure R stereoisomer (most preferably R,R stereoisomer) of the dopamine agonist is preferably in the form of a pharmaceutically acceptable salt, preferably included in a dosage form with a suitable carrier.

EP 0 404 115 A1

## BIS(BENZYLPYRROLIDINE) DERIVATIVES AS DOPAMINE AGONISTS

The invention relates generally to dopamine agonists and more particularly to bis(benzylpyrrolidine) derivatives which are orally active dopamine agonists and as such are useful in the treatment of hypertension, congestive heart failure and acute renal failure in mammals. The invention also relates to methods of making such compounds, pharmaceutical dosage forms comprising such compounds and to methods of treatment involving the administration of such compounds to a mammal.

Drugs having the pharmacological effect of dopamine are referred to as dopaminergic agonists and dopamine is a marketed sympathomimetic with significant dopaminergic actions in the periphery; some sympathomimetics appear to act on dopamine receptors in the central nervous system. In the periphery, dopamine receptors are prominent in the splanchnic and renal vascular beds, where they mediate vasodilation. Dilation in these beds is important in the treatment of shock and acute heart failure, since these beds are often critically constricted in these conditions. Dopamine is used in the management of these disorders. It may also be used to induce diuresis, probably consequent to renal vasodilatation, at least in part.

DOPAMINE HYDROCHLORIDE

1,2-Benzenediol,4-(2-aminoethyl-, hydrochloride; Intropin (Arnar-Stone)

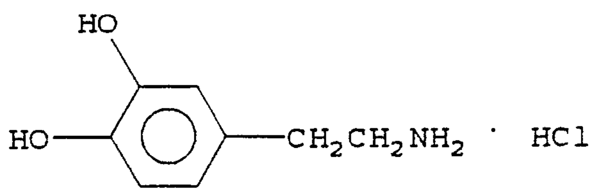

3,4-Dihydroxyphenethylamine Hydrochlorid $C_8H_{11}NO_2HCl$ (189.64)

### Preparation

Dopamine, which is 3-hydroxytyramine, may be prepared from tyramine by successive nitration to 3-nitrotyramine, reduction to 3-aminotyramine by catalytic hydrogenation, and diazotization to 3-hydroxytyramine.

### Description

Dopamine hydrochloride occurs as a white, crystalline powder; decomposes at about 241 °C. To avoid oxidation of dopamine hydrochloride injection, the air in containers is replaced with nitrogen. Yellow or brown discoloration of solutions indicates decomposition of the drug, and such solutions should not be used.

### Solubility

Freely soluble in water; soluble in alcohol; practically insoluble in chloroform and ether.

**Uses**

Dopamine is a natural catecholamine formed by the decarboxylation of 3,4-dihydroxyphenylalanine (DOPA). It is a precursor to norepinephrine in noradrenergic nerves and is also a neurotransmitter in certain areas of the central nervous system, especially in the nigrostriatal tract, and in a few peripheral sympathetic nerves.

In the central nervous system and the mesenteric, coronary and renal vascular beds, it acts upon dopamine receptors that are distinct from alpha- and beta-adrenoreceptors. At these dopamine receptors, haloperidol is an antagonist. In the above-named vascular beds it causes vasodilatation. The renal vasodilatation may be one stimulus for diuresis. Dopamine also has moderate $beta_1$- and weak alpha-agonist activities, part of which is attributable to norepinephrine released by dopamine. During a low rate of intravenous infusion, only vasodilatation in the mesenteric, coronary and renal vascular beds usually predominates, and hypotension sometimes occurs. At an intermediate rate of infusion, the heart rate and force of contraction are increased, as is cardiac output, and blood pressure may increase accordingly. At high rates of infusion, alpha-adrenergic vasoconstriction in the mesenteric, coronary and renal vascular beds may overcome the dopaminergic vasodilatation in some recipients.

Dopamine is used in the treatment of shock, for which it has several advantages. Firstly, vasodilatation can often be effected in the two organs most likely to suffer ischemic damage in shock (kidney and small bowel); blood may be moved from the skeletal muscle to more vital organs, cardiac stimulation improves a usually deteriorated cardiac function, and diuresis also helps to preserve renal function. Although dopamine is now the vasopressor agent of choice in shock, a substantial fraction of cases nevertheless fail to respond. Dopamine is also used to treat acute heart failure; the decreased vascular resistance decreases the cardiac afterload, the cardiostimulatory actions improve cardiac output, and the diuresis lessens edema.

U.S. Patent 4,613,606 issued September 23, 1986 discloses a number of tetrahydroisoquinoline derivatives which are indicated as being calcium channel blockers and as such useful for the treatment of cardiovascular disorders including angina, hypertension and congestive heart failure.

European Patent Application No. 0,294,973 published December 14, 1988 discloses a number of dopamine-beta-hydroxylase inhibitors (DBH inhibitors). Dopamine is hydroxylated to norepinephrine by (DBH) in the presence of oxygen and ascorbic acid. There are a number of known DBH inhibitors discussed in 0,294,973 which are believed to be effective in treating hypertension. Methods of synthesizing the novel compounds are also disclosed.

A primary object of the present invention is to disclose and provide novel compounds represented by the formula

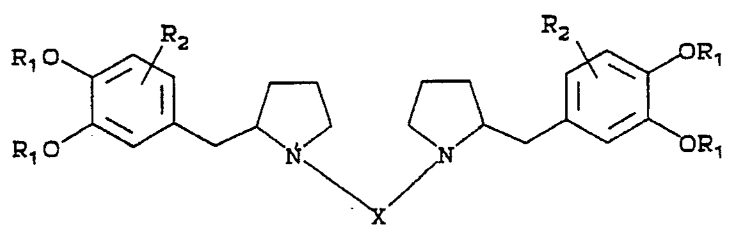

(I)

in which $R_1$ is independently hydrogen, $-C(O)R_3$, or $-C(O)NR_3R_4$,

wherein:

$R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;

$R_2$ is hydrogen or lower alkyl;

X is $-(CH_2)_m-$ or $-(CO_2)_nY(CH_2)_n-$, where m is an integer from 1 to 10, n is an integer of 1 to 3 and Y is oxygen or sulfur;

and pharmaceutically acceptable salts or esters thereof.

Another object is to provide racemic and non-racemic mixtures of the compounds of formula (I) which include R,R; R,S and S,S optically pure compositions.

Another object of the invention is to disclose and provide novel methods of preparing the compounds of formula (I) and their salts and esters as well as the novel intermediates produced by such methods. For example, a process for the preparation of compounds of formula I:

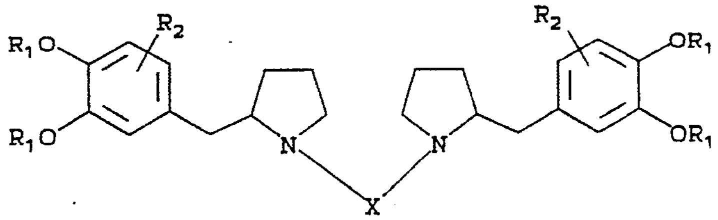

(I)

wherein:

$R_1$ is independently hydrogen, lower alkyl, $-C(O)R_3$, $-C(O)NR_3R_4$, wherein $R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;

$R_2$ is hydrogen or lower alkyl;

X is $-(CH_2)_m-$ or $-(CH_2)_nY(CH_2)_n-$, where m is an integer from 1 to 10, n is an integer of 1 to 3 and Y is oxygen or sulfur;

or a pharmaceutically acceptable salt or ester thereof, which comprises

a) reacting a compound of formula II

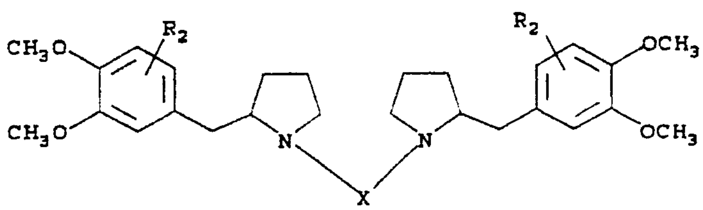

(II)

wherein $R_2$ and X are as defined above with a deprotecting agent; or

b) converting a compound of formula I to a pharmaceutically acceptable salt of formula I; or

c) converting a pharmaceutically acceptable salt of formula I to a free compound of formula I; or

d) converting a pharmaceutically acceptable salt of formula I to another pharmaceutically acceptable salt of formula I; or

e) converting a compound of formula I to an ester of formula I.

Yet another object is to disclose and provide pharmaceutical compositions and dosage forms containing compounds of formula (I) and their salts and esters with pharmaceutically acceptable non-toxic carriers. For example, a composition, comprising:

a pharmaceutically acceptable carrier; and

a compound of formula (I) including mixtures of optically active compounds and optically pure R and S steroisomers:

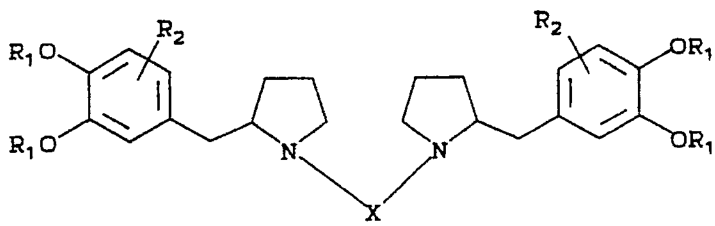

(I)

wherein:

$R_1$ is independently hydrogen, $-C(O)R_3$, $-C(O)NR_3R_4$, wherein $R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;

$R_2$ is hydrogen or lower alkyl;

X is $-(CH_2)_m-$ or $-(CH_2)_nY(CH_2)_n-$, where m is an integer from 1 to 10, n is an integer of 1 to 3 and Y is

oxygen or sulfur;
or a pharmaceutically acceptable salt or ester thereof.

Still another object of the invention is to disclose and provide methods of treating hypertension, congestive heart failure and acute renal failure in mammals by administering to the mammals the dosage forms of the invention. For example, a method of treating hypertension in a mammal, comprising administering to the mammal in need of treatment a pharmaceutically effective amount of a composition comprising:
a pharmaceutically acceptable carrier; and
a compound of formula (I) including racemic mixtures of optically active compounds and optically pure R and S stereoisomers:

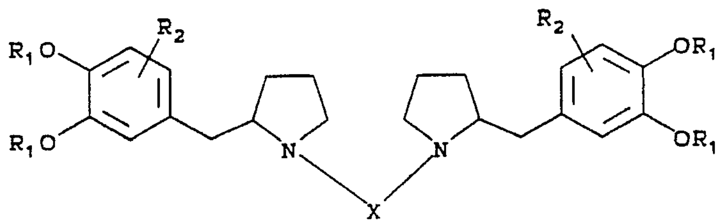

(I)

wherein:
$R_1$ is indepedently hydrogen, $-C(O)R_3$, $-C(O)NR_3R_4$, wherein $R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;
$R_2$ is hydrogen or lower alkyl;
X is $-(CH_2)_m-$ or $-(CH_2)_nY(CH_2)_n-$, where m is an integer from 1 to 10, n is an integer of 1 to 3 and Y is oxygen or sulfur;
or a pharmaceutically acceptable salt or ester thereof.

Another object of the invention are compounds of the formula:

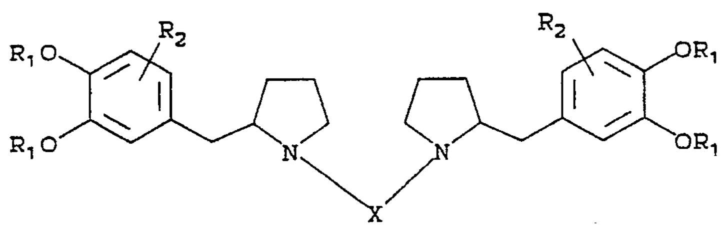

(I)

wherein:
$R_1$ is lower alkyl, $-C(O)R_3$, $-C(O)NR_3R_4$, wherein $R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;
$R_2$ is hydrogen or lower alkyl;
X is $-(CH_2)_m-$ or $-(CH_2)_nY(CH_2)_n-$, where m is an integer from 1 to 10, n is an integer of 1 to 3 and Y is oxygen or sulfur;
or a pharmaceutically acceptable salt or ester thereof.

A feature of the present invention is that compounds of formula (I) (particularly the R and R,R stereoisomers) have oral bioavailability.

Another feature of the present invention is that the compounds of formula (I) can be produced via an efficient process and that processes disclosed allow for the production of mixtures and optically pure stereoisomers.

An advantage of the present invention is that the compounds of formula (I) are very effective dopamine agonists.

An important advantage of the compounds of this invention is that they can be administered orally to

treat hypertension and congestive heart failure.

These and other objects, advantages and features of the present invention will become apparent to those persons skilled in the art upon reading the details of the various compounds, salts and esters thereof, methods of synthesis, and usage as more fully set forth below. In the following description, reference is made to the accompanying general structural formulae designated by Roman numerals, numbers or letters wherein like symbols refer to like molecular moieties throughout.

## Definitions

"Alkyl" means a branched or unbranched saturated hydrocarbon chain containing 1 to 10 carbon atoms, such as methyl, ethyl, propyl, iso-propyl, butyl, iso-butyl, tert-butyl, n-pentyl, n-hexyl, 2-methylheptyl, n-octyl and the like, unless otherwise indicated.

"Lower alkyl" means a branched or unbranched saturated hydrocarbon chain containing 1 to 4 carbon atoms, such as methyl, ethyl, propyl, isopropyl, tert-butyl, butyl, iso-butyl and the like, unless otherwise indicated.

"Lower alkoxy" means the group -OR wherein R is lower alkyl as herein defined.

"Halo" as used herein denotes fluoro, chloro, bromo, or iodo, unless otherwise indicated.

"Phenyl" as used herein encompasses all possible isomeric phenyl radicals optionally monosubstituted or disubstituted with a substituent selected from the group consisting of lower alkyl, lower alkoxy, hydroxy, trifluoromethyl and halo.

"Phenyl-lower-alkyl" as used herein denotes phenyl as defined herein attached to a lower alkyl group as defined herein.

"Optional" or "optionally" means that the subsequently described event or circumstance may or may not occur, and that the description includes instances where said event or circumstance occurs and instances in which it does not. For example, "optionally substituted phenyl" means that the phenyl may or may not be substituted and that the description includes both unsubstituted phenyl and substituted phenyl.

"Protecting group" means any suitable chemical group that is commonly used in the practice of organic chemistry to modify one or more of the major functional groups in a molecule for the purpose of selectively performing a chemical reaction at another reactive site in a multifunctional molecule. A protecting group is typically formed in a selective manner and is stable to subsequent reactions on the molecule and is selectively removed by reagents that do not attack the regenerated functional group. Suitable protecting groups for the amino group are carbamates such as methyl carbamates and its derivatives like cyclopropylmethyl, diisopropylmethyl, 9-fluorenylmethyl carbamates and the like, substituted ethyl carbamates such as 2,2,2-trichloroethyl, 2-haloethyl, and the like, substituted propyl and isopropyl carbamates such as 1,1-dimethylpropynyl, 1-methyl-1-phenylethyl and derivatives, isobutyl, t-butyl carbamate, t-amyl carbamate, vinyl and allyl carbamate, phenyl and substituted phenyl carbamate, benzyl carbamate and derivatives such as p-methoxybenzyl, 3,5-dimethoxybenzyl, o- and p-nitrobenzyl, halobenzyl, and the like; amides and their derivatives such as N-acetyl and derivatives like N-dichloroacetyl, N-trifluoroacetyl, and the like, substituted N-propionyl derivatives such as N-3-phenylpropionyl and derivatives, N-o-nitrocinnamoyl and the like, cyclic imide derivatives such as N-phthaloyl, N-2,3-diphenylmaleoyl, and the like.

A "leaving group" means a group capable of being displaced by a nucleophile in a chemical reaction, for example chloro, bromo, iodo, sulfonate ester, sulfinate ester, carbamate and the like.

The term "pharmaceutically acceptable salts" refers to salts of the subject compounds which possess the desired pharmacological activity and which are neither biologically nor otherwise undesirable. These salts are formed with inorganic acids such as hydrochloric acid, hydrobromic acid, sulfuric acid, nitric acid or phosphoric acid; or organic acids such as acetic acid, propionic acid, glycolic acid, pyruvic acid, malonic acid, succinic acid, malic acid, maleic acid, fumaric acid, tartaric acid, citric acid, benzoic acid, ethanesulfonic aicd, p-toluenesulfonic acid and the like.

"Esters" are those compounds of formula I in which $R_1$ is $-C(O)R_3$ wherein $R_3$ is lower alkyl, optionally substituted phenyl or optionally substituted phenyl lower alkyl.

The compounds of this invention possess asymmetric centers and thus can be produced as mixtures of stereoisomers or as individual stereoisomers. The individual stereoisomers may be obtained by using an optically active starting material, by resolving a racemic or non-racemic mixture of an intermediate at some appropriate stage of the synthesis or by resolution of the compound of formula (I). It is understood that the individual stereoisomers as well as mixtures (racemic and non-racemic) of stereoisomers are encompassed by the scope of the present invention. The compounds of this invention possess at least two asymmetric centers and thus can be produced as mixtures of stereoisomers or as individual R,R, S,S or R,S

stereoisomers. The individual enantiomers may be obtained by resolving a racemic or non-racemic mixture of an intermediate at some appropriate stage of the synthesis. It is understood that the individual R,R, S,S or R,S stereoisomers as well as mixtures of stereoisomers are encompassed by the scope of the present invention. The R,R stereoisomer is most preferred due to its greater activity especially when administered orally.

"Isomers" are different compounds that have the same molecular formula.

"Stereoisomers" are isomers that differ only in the way the atoms are arranged in space.

"Enantiomers" are a pair of stereoisomers that are non-superimposable mirror images of each other.

"Diastereoisomers" are stereoisomers which are not mirror-images of each other.

"Epimers" are diastereoisomers which differ only in the configuration of one asymmetric center.

"Meso" isomers are diastereoisomers that are optically inactive by reason of internal compensation. That is, the compounds of formula (I) where one of the two asymmetric carbon atoms is R and the other is S are meso compounds, because equal and opposite optical rotations are thus produced.

"Racemic mixture" means a mixture containing equal parts of individual enantiomers. "Non-racemic mixture" is a mixture containing unequal parts of individual enantiomers or in the case of this disclosure, stereoisomers.

The term "treatment" as used herein covers any treatment of a disease and/or condition in a mammal, particularly a human, and includes:

(i) preventing a disease and/or condition from occurring in a subject which may be predisposed to the disease and/or condition but has not yet been diagnosed as having it;

(ii) inhibiting the disease and/or condition, i.e., arresting its development; or

(iii) relieving the disease and/or condition, i.e., causing regression of the disease and/or condition.

The system used in naming the intermediates and product compounds of the present invention is shown below, using a compound of formula (I) as an example.

A racemic compound of formula (I) wherein $R_1$ and $R_2$ are hydrogen and X is $(CH_2)_m$- where m is 7 is named:

(±)-1,7-bis-[2-(3,4-dihydroxybenzyl)pyrrolidyl]-heptane.

A racemic compound of formula (I) wherein $R_1$ and $R_2$ are hydrogen and X is $-(CH_2)_nY(CH_2)_n-$, where n is 2 and Y is sulfur is named:

(±)-2,2-bis-[2-(3,4-dihydroxybenzyl)pyrrolidyl]-di-ethyl thioether.

A racemic compound of formula (I) wherein $R_1$ is $-C(O)CH_3$, $R_2$ is 5-methyl, and X is $-(CH_2)_m-$, where m is 4 is named:

(±)-1,4-bis-[2-(3,4-diacetoxy-5-methylphenyl methyl)pyrrolidyl]butane.

Before the present dopamine agonist compounds and processes for making and using pharmaceutical compositions containing such are described, it is to be understood that this invention is not limited to the particular compounds, intermediates and processes described as such and may, of course, vary. It is also to be understood that the terminology used herein is for the purpose of describing particular embodiments only, and is not intended to be limiting since the scope of the present invention will be limited only by the appended claims.

It must be noted that as used in this specification and the appended claims, the singular forms "a", "an" and "the" include plural reference unless the context clearly dictates otherwise. Thus, for example, reference to a bis(benzylpyrrolidyl) derivative includes mixtures of such compounds, reference to "the hydrogenating reaction" includes reference to a plurality of hydrogenating reactions, reference to "an ester" includes mixtures of esters and so forth.

The following examples are put forth so as to provide those of ordinary skill in the art with a complete disclosure and description of compounds and compositions of the invention, how to carry out the processes and use the compounds and compositions of the invention and are not intended to limit the scope of what the inventors regard as their invention. Unless indicated otherwise, parts are parts by weight, temperature is in degrees C and is at room temperature (20° C to 30°) and pressure is at or near atmospheric.

Methods of Preparation

One method of preparing racemic compounds of formula (I) is illustrated below in Reaction Scheme I.

REACTION SCHEME I

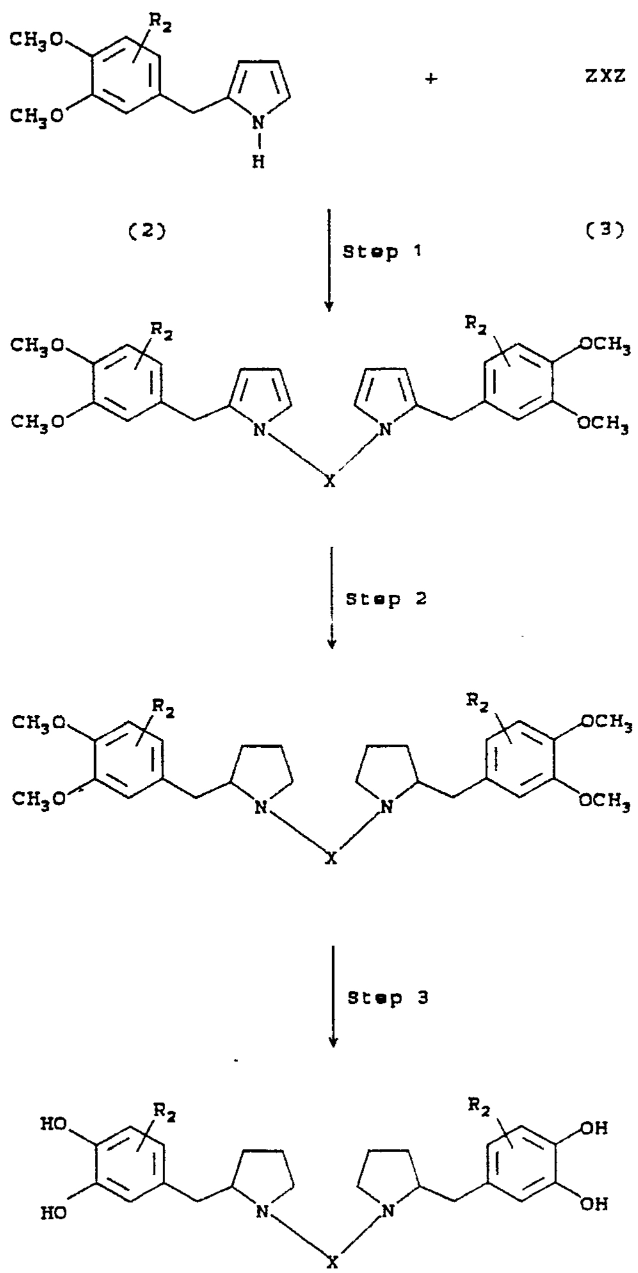


(2) (3) (4) (5) (6)

Where $R_2$ and X are as defined above. $R_2$ is preferably hydrogen and each Z is a leaving group such as a halo or sulfonyl and ZXZ is most preferably BrXBr with X being $(CH_2)_m$ and m is 4. When $R_2$ is hydrogen X is preferably $-(CH_2)_m-$ and m is most preferably an integer of from 4 to 8.

In the Reaction Scheme I above $R_1$ is $-CH_3$ in steps 1 and 2 and changes to -OH in step 3. No stereoisomerism is indicated throughout. However, steroisomerism is introduced in Step 2 and is found in both compounds (5) and (6). Both the right and left sides of the compound have an asymmetric center and as such either side may be R or S. The most preferred compounds are R,R and the least preferred are S,S.

REACTION SCHEME 1

## A GENERAL DESCRIPTION OF STEPS 1-3

### STEP 1

The preferred method of preparing the compounds of formula (I) as a racemic mixture starts from the compound of formula (2). The compound (2), which is known as 2-(3,4-dimethoxybenzyl)pyrrole, can be synthesized by methods known to those skilled in the art such as by the sodium borohydride reduction of 2-(3,4-dimethoxybenzoyl) pyrrole as described by R. Greehouse, et. al., J. 0rg. Chem., 50, 2961 (1985). The synthesis of the 2-(3,4-dimethoxybenzoyl)pyrrole is also described in the Greenhouse et. al. article which is incorporated herein by reference to disclose the synthesis of such compounds and the compound (2).

Typically 2-(3,4-dimethoxybenzyl)pyrrole is dissolved in a polar solvent such as dimethylsulfoxide, sulfolane and the like, preferably dimethylformamide. The solution is added to a suspension of 1 to 2 molar equivalents, preferably about 1.2 molar equivalents, of a metal hydride such as potassium hydride, lithium hydride, preferably sodium hydride, preferably in the same polar solvent. The reaction is carried out at a temperature of about 0 to 30°C, preferably about 25°C, for about 30 minutes to 6 hours, preferably about 2 hours. To this reaction mixture is then added ZXZ, the compound of formula (3), where X is as defined above and Z is a leaving group, for example methanesulfonyl, p-toluenesulfonyl, iodo, chloro and the like, preferably bromo (Z is preferably Br and X is most preferably $(CH_2)_6$. The reaction is carried out at a temperature in the range of about 0 to 30°C, preferably about 25°C, for about 1 to 8 hours, preferably about 3 hours. When the reaction is substantially complete, the product of formula (4) is isolated and purified by conventional means, preferably chromatography.

### STEP 2

In STEP 2 the pyrrole of formula (4) is reduced to the pyrrolidyl compound of formula (5). Typically the compound of formula (4) is dissolved in an organic acid solvent, preferably acetic acid, and a hydrogenation catalyst added, preferably 5% rhodium on alumina. The mixture is reacted with hydrogen at about 20 to 80 p.s.i, preferably about 45 p.s.i at a temperature of about 0 to 30°C, preferably about 25°C, until about the theoretical amount of hydrogen is absorbed. When the reaction is substantially complete, the product of formula (5) is isolated by conventional means, preferably chromatography.

### STEP 3

A solution of the compound 5 in dry $CH_2Cl_2$ is cooled to -70°C under inert atmosphere and $BBr_3$ is added. The mixture is warmed to room temperature and $CH_3OH$ is added. The solvent is removed *in vacuo* to give the bis-catechol compound 6, which is also compound I.

Pharmaceutically acceptable salts of the compounds of formula 6 or I are prepared by reacting a free base of formula 6 or I with an appropriate acid. Free bases of formula 6 or I are prepared by reacting a salt of a compound of formula 6 or I with an appropriate base.

If desired, compounds of formula I prepared using the processes of Reaction Scheme I may be resolved into individual stereoisomers using resolution techniques known in the art.

If desired, compounds of formula I wherein $R_1$ is $-C(O)R_3$ or $-C(O)NR_3R_4$ wherein $R_1$, $R_3$ and $R_4$ are as defined above may be prepared.

## REACTION SCHEME II

One method of preparing the optically active isomers of the compounds of formula (I) is shown in Reaction Scheme II.

## REACTION SCHEME II

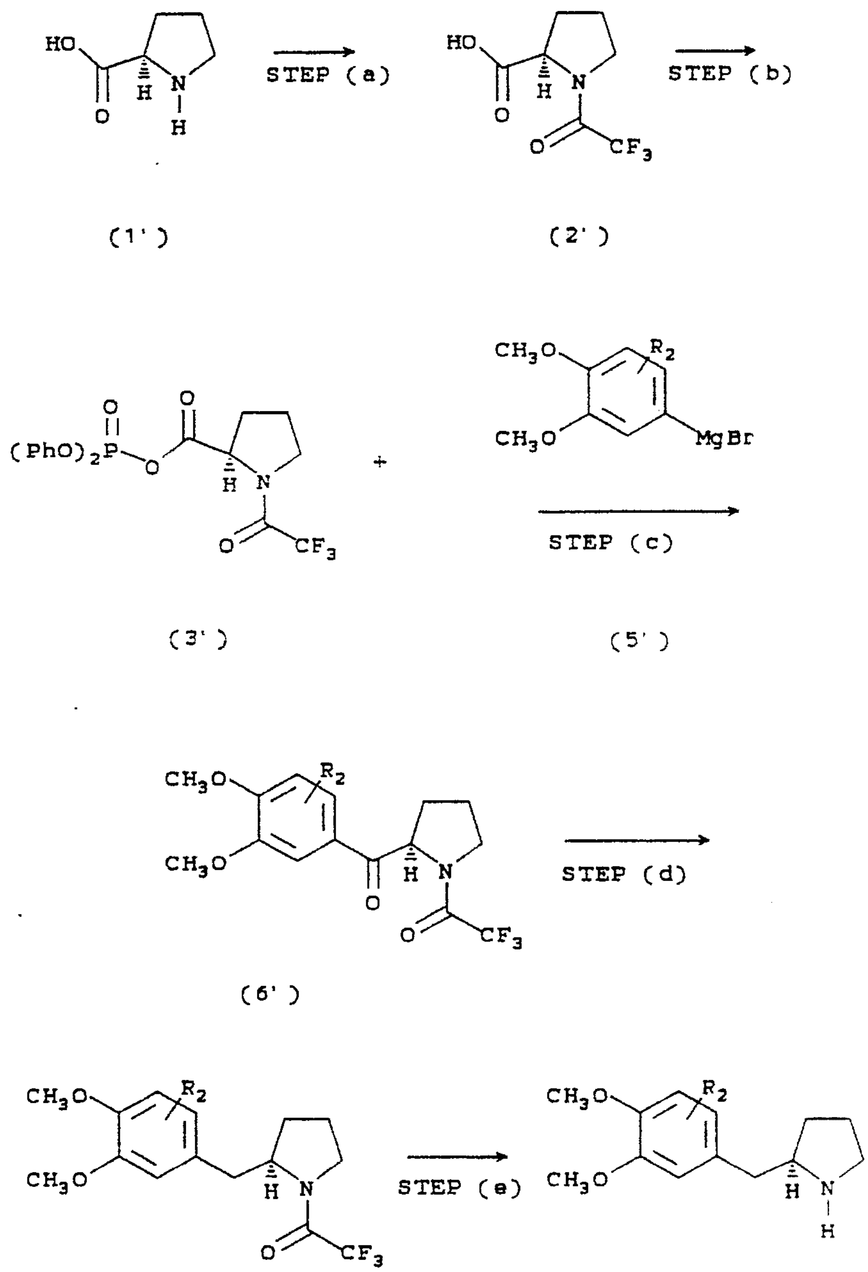


(1') (2') (3') (5') (6') (7') (8')

(8')

bis-acid chloride

STEP (f)

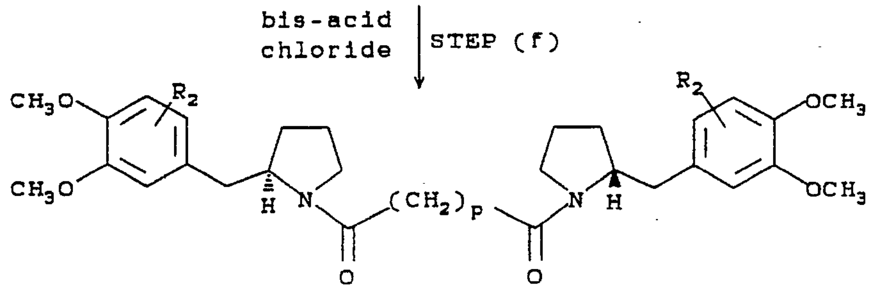

(10')

STEP (g)

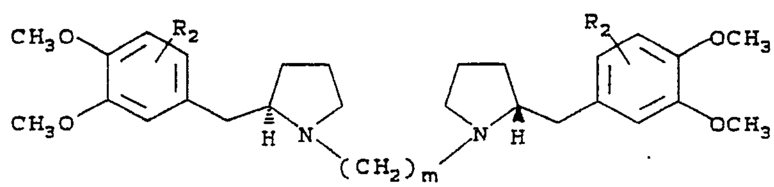

(11')

STEP (h)

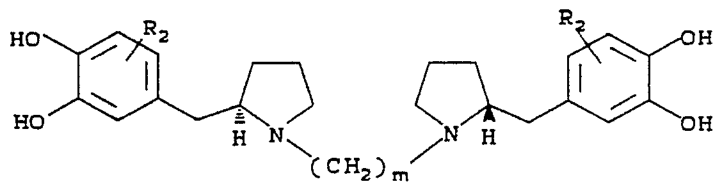

(I)

(Note: (I) is the R,R stereoisomer most preferred.) where p = m-2 (but cannot be less than 0).

## GENERAL DESCRIPTION OF REACTION SCHEME II STEPS

The preferred method for the preparation of the compounds of formula (I) as their optical isomers where $R_2$ is hydrogen starts from commercially available optically pure R- or S-proline.

### STEP(a)

STEP (a) involves the protection of the amine function of R- or S-proline, preferably as the

trifluoroacetyl derivative. Typically, the amine of formula (1′) is reacted, in an inert solvent such as benzene, toluene, acetonitrile, diethyl ether, chloroform, methylene chloride or preferably tetrahydrofuran, with from 1 to 5 molar equivalents, preferably about 2 molar equivalents, of a trifluoroacetylating agent, preferably ethyl trifluoroacetate, in the presence of about 1.0 to 3 molar equivalents, preferably about 1.5 molar equivalents, of a tertiary organic base, such as pyridine, N-methylpiperidine, 4-dimethylaminopyridine and the like, preferably 1,1,3,3-tetramethylguanidine. The reaction is carried out at a temperature of about 0 to 40° C, preferably about 25° C, for about 10 minutes to 4 hours, preferably about 30 minutes. When the reaction is substantially complete, the product of formula (2′), a 1-trifluoroacetyl- R- or S-proline is isolated by conventional means.

STEP (b)

In STEP (b) the carboxyl group of the protected amine of formula (2′) is converted to a derivative which on reaction with an organometallic compound gives a ketone. Methods for converting carboxyl groups to ketones are well known in the art, and include converting the carboxyl group to an acid halide and reacting this with a lithium dialkylcopper reagent or an organocadmium reagent, or converting the carboxyl group to a tertiary amide and reacting this with a Grignard reagent or organolithium derivative. Such reactions are discussed in more detail in Advanced Organic Chemistry by March, for example on pages 439-440 (2nd Edition), the pertinent portions of which are hereby incorporated by reference. The preferred method is to convert the carboxyl group to a mixed anhydride with diphenylphosphoric acid, most preferably to a R- or S-2-carboxy-1-trifluoroacetyl pyrrolidine anhydride with diphenylphosphoric acid. Typically the protected amine of formula (2′) is dissolved in an inert solvent as defined above, preferably methylene chloride, and reacted with from 0.5 to 1.5 molar equivalents, preferably about 1 molar equivalent, of an organochlorophosphate, preferably diphenyl chlorophosphate, in the presence of about 1.0 to 3 molar equivalents, preferably about 1.5 molar equivalents, of a tertiary organic base, such as pyridine, N-methylpiperidine and the like, preferably N-methylmorpholine. The reaction is carried out at a temperature of about -20 to 20° C, preferably about 0° C, for about 5 minutes to 1 hour, preferably about 10 minutes followed by a temperature of about 0 to 30° C, preferably about 25° C, for about 5 minutes to 1 hour, preferably about 10 minutes. When the reaction is substantially complete, the product of formula (3′) is isolated by conventional means. The R- or S-2-carboxy-1-trifluoroacetylpyrrolidine anhydride with diphenylphosphoric acid of formula (3′) is hygroscopic and unstable to heat and moisture, and is therefore preferably used in the next step without delay.

STEP (c)

In STEP (c) the R- or S-2-carboxy-1-trifluoroacetyl pyrrolidine anhydride with diphenylphosphoric acid of formula (3′) is converted to a ketone of formula (6′). Typically the compound of formula (3′) is dissolved in an inert solvent as defined above, preferably tetrahydrofuran, and cooled to a temperature of about -100 to -50° C, preferably about -60° C to -70° C. To the cold solution is added from 1 to 2 molar equivalents, preferably about 1.1 molar equivalents, of a Grignard reagent of formula (5′) formed from commercially available 4-bromoveratrole at such a rate such that the temperature is maintained within the preferred range. The reaction mixture is then allowed to rise to a temperature of about 0 to 30° C, preferably about 25° C, for about 5 to 30 hours, preferably about 14 hours. When the reaction is substantially complete, the product of formula (6′), R- or S- 2-(3,4-dimethoxybenzoyl)-1-trifluoroacetylpyrrolidine, is isolated and purified by conventional means, preferably chromatography.

STEP (d)

In STEP (d) the ketone of formula (6′) is reduced to the compound of formula (7′), R- or S- 2-(3,4-dimethoxybenzyl)-1-trifluoroacetylpyrrolidine. Typically the compound of formula (6′) is dissolved in an inert solvent as defined above, preferably methylene chloride. To the solution is added from 5 to 20 molar equivalents, preferably about 11 molar equivalents, of a reducing agent, preferably triethylsilane/boron

trifluoride etherate mixture . The reaction is carried out at a temperature of about 0 to 30° C, preferably about 25° C, for about 1 to 7 days, preferably about 3 days. When the reaction is substantially complete, the product of formula (7′) is isolated by conventional means.

STEP (e)

In STEP (e), the amine protecting group is removed from the compound of formula (7′). Typically the compound of formula (7′) is dissolved in a protic solvent such as ethanol, n-propanol, n-butanol, t-butanol and the like, preferably isopropanol, and to the solution is added from 5 to 50 molar equivalents, preferably about 20 molar equivalents, of an acid, for example sulfuric acid, HBr and the like, or preferably 12.5 M hydrochloric acid. The reaction is carried out at the reflux temperature of the solvent chosen, preferably about 70 to 90° C, for about 8 to 48 hours, preferably about 24 hours. When the reaction is substantially complete, the product of formula (8′), R- or S-2-(3,4-dimethoxybenzyl)pyrrolidine, is isolated by conventional means.

STEP (f)

In STEP (f ), two equivalents of the compound of formula (8′) are reacted with one equivalent of a bis acid chloride to give a compound of formula (10′). The alkylene linking group of the bis acid chloride can contain from 1 to 10 carbons, preferably 2 to 8 carbons and more preferably 3 to 6 carbons. Typically the compound of formula (8′) is dissolved in an inert solvent as defined above, preferably methylene chloride, and to the solution is added from 0.5 to 0.9 molar equivalents, preferably about 0.67 molar equivalents, of a bis acid chloride of formula $ClC(O)(CH_2)_pC(O)Cl$, in which p is equal to m-2, where m is as defined above, but p cannot be less than 0. The mixture is cooled to a temperature of about -20 to 10° C, preferably about 0° C. To the cold solution is added from 1 to 10 molar equivalents, preferably about 3 molar equivalents, of a tertiary organic base, such as pyridine, N-methylpiperidine and the like, preferably triethylamine. The temperature of the reaction mixture is then allowed to rise to a temperature of about 0 to 30° C, preferably about 25° C, for about 30 minutes to 4 hours, preferably about 1 hour. When the reaction is substantially complete, the product of formula (10′) is isolated by conventional means.

STEP (g)

In STEP (g) a reduction reaction is carried out by any procedure generally known to those skilled in the art. For example add 0.001 moles of (10′) in dry THF to a solution containing 0.5 ml(0.005 moles) 10 M $BH_3$.DMS and THF. Heat under reflux in an atmosphere of argon. Continue heating for 2 hours, cool and add 10 ml of anhydrous methanol dropwise. Add 10ml of saturated HCl-MeOH dropwise and heat for 15 minutes. Cool to room temperature and remove the solvent in vacuo, leaving an oily residue. Dissolve the residue in isopropyl alcohol and ether. Product (11′) will crystallize out overnight.

STEP (h)

In STEP (h) follow the general procedure of STEP 3 or the more specific procedure of STEP 3A below to obtain the product (I). In (I) the optically pure form R,R of the compound of the invention is shown. The R,R compound (I) provides the best oral activity.

The compounds of formula (I) where $R_2$ is not hydrogen are prepared as shown in Reaction Scheme II above, starting with the compound of formula (1′) and using a reactant of formula (5′) which is optionally substituted with an appropriate definition of $R_2$, and reacting the compound of formula (3′) with the compound of formula (5′).

It is clear that the racemic compounds of formula (I) could be also prepared by the method shown in Reaction Scheme II for the preparation of the optically pure compounds of formula (I). However, the

preferred method of making non-optically pure mixtures, i.e. racemic and non-racemic mixtures is as shown in Reaction Scheme I.

It should be noted that optically pure compounds of the invention can be obtained by carrying out a reaction scheme (such as REACTION SCHEME I) which produces a racemic mixture and then carrying out resolution of the optically pure compounds from the mixture by conventional means. However, the preferred method of making optically pure compounds is as shown in Reaction Scheme II in a manner so as to obtain the most preferred stereoisomers R,R.

## UTILITY AND ADMINISTRATION

The compounds of Formula I and the pharmaceutically acceptable non-toxic esters and salts thereof, are useful in the treatment of hypertension and congestive heart failure in mammals. These compounds can be used both prophylactically and therapeutically.

Pharmaceutical dosage forms which include compositions containing compounds of formula (I) and salts thereof are thus administered to patients suffering from hypertension or congestive heart failure. The compounds act to relieve blood pressure and improve heart action by acting as dopamine agonists. In addition, these compositions may be used to treat other conditions as recognized by those skilled in the art.

Compounds of the present invention can be used to prepare pharmaceutical compositions useful in the treatment of hypertension and congestive heart failure in mammals comprising a therapeutically effective amount of a compound of formula I, or a pharmaceutically acceptable salt or ester thereof, in admixture with a pharmaceutically acceptable non-toxic carrier. A therapeutically effective amount is that amount which, when administered to a mammal in need thereof, is sufficient to effect treatment, as defined above. Thus, the level of the drug in the formulation can vary from about 1 percent weight (%w) to about 99%w of the drug based on the total formulation and about 1%w to 99%w excipient.

The compounds of the invention can be administered by any suitable means but are usually administered by I.V. or orally. The oral administration of the R,R stereoisomer is most preferred and is given in an amount of 0.1 to 500 mg per kilogram of body weight preferrably 3 to 30 mg per kilogram of body weight. The amount can be adjusted over a wide range depending on the needs and condition of the mammal.

Useful pharmaceutical carriers for the preparation of the pharmaceutical compositions hereof can be solids or liquids. Thus, the compositions can take the form of tablets, pills, capsules, powders, sustained release formulations, solutions, suspensions, elixirs, aerosols, and the like. Carriers can be selected from the various oils, including those of petroleum, animal, vegetable or synthetic origin, for example, peanut oil, soybean oil, mineral oil, sesame oil, and the like. Water, saline, aqueous dextrose, and glycols are preferred liquid carriers, particularly for injectable solutions. Suitable pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. Other suitable pharmaceutical carriers and their formulations are described in "Remington's Pharmaceutical Sciences" by E. W. Martin.

One aspect of the present invention is a method for treating congestive heart failure or hypertension in a mammalian subject (particularly a human) which method comprises administering a therapeutically effective amount of a compound of formula I, or a pharmaceutically acceptable salt or ester thereof, to a mammal in need thereof.

In the practice of the above described method of the present invention a therapeutically effective amount of the compound of formula I or a pharmaceutical composition containing same is administered via any of the usual and acceptable methods known in the art, either singly or in combination with another compound or compounds of the present invention or other pharmaceutical agents. These compounds or compositions can thus be administered orally, systemically (e.g., transdermally, intranasally or by suppository) or parenterally (e.g., intramuscularly, subcutaneously and intravenously), and can be administered either in the form of solid or liquid dosages including tablets, solutions, suspensions, aerosols, and the like, as discussed in more detail above.

The formulation can be administered in a single unit dosage form for continuous treatment or in a single unit dosage form ad libitum when relief of symptoms is specifically required.

## EXPERIMENTAL DETAILS OF REACTION STEPS

The following provides additional specific information on the STEPS of REACTION SCHEME I and REACTION SCHEME II. Unless indicated otherwise, parts are parts by weight, temperature is in degrees C and is at room temperature (20°C to 30°) and pressure is at or near atmospheric.

## REACTION SCHEME I

## DETAILED DESCRIPTION OF STEPS 1-3

### STEP 1

1,6-Bis{2-(3,4-dimethoxybenzyl)pyrrol-1-yl}hexane

A solution of 2-(3,4-dimethoxybenzyl)pyrrole (2.5 g, 11.5 mmol) in dry DMF (40ml) was added to a stirred suspension of 50% sodium hydride dispersed in mineral oil (0.663 g, 13.8 mmol) in dry DMF (5 ml) at room temperature (nitrogen atmosphere). After 2 hours, 1,6-dibromohexane (1.24 g, 5.7 mmol) was added dropwise (during which time the reaction temperature rose to about 50°C. The solution was stirred for 3 h at room temperature (by which time the reaction was complete as judged by TLC {hexane-ethyl acetate (4:2)}. The solution was poured into an ice-water mixture and the product extracted into ethyl acetate (3 x 100 ml). The extract was washed with saturated sodium chloride solution (5 x 100 ml), dried over sodium sulfate and evaporated in vacuo. The residue was subjected to column chromatographic purification on silica gel (150 g) the product being eluted with the solvent system mentioned above. A crystalline solid was obtained by a crystallization from dichloromethane-hexane giving the product 1,6-Bis{2-(3,4-dimethoxybenzyl)pyrrol-1-yl} hexane.

### STEP 2

1,6-Bis{2-(3,4-dimethoxybenzyl)pyrrolidin-1-yl}hexane

A solution of 1,6-Bis{2-(3,4-dimethoxybenzyl)pyrrol-1-yl}hexane (2.5 g) in glacial acetic acid (30 ml) containing suspended 5% Rh on alumina catalyst (1.87 g) was reacted at room temperature and an initial pressure of 45 p.s.i.g. (3 hr reaction followed by TLC). The mixture was filtered through Celite. The filtrate was dried at reduced pressure. The residue was placed in dichloromethane, washed with ammonium hydroxide solution and dried over sodium sulfate. The solvent was removed in vacuo and the residue subjected to column chromatographic purification on silica gel (100 g) using the dichloromethane mixture to elute the product 1,6-Bis{2-(3,4-dimethoxybenzyl)pyrrolidin-1-yl}hexane, 2.5 g, 92%, oil).

### STEP 3

2-(3,4-dimethoxybenzyl)pyrrolidine hexane was dissolved in dry $CH_2Cl_2$ and cooled to -70°C under inert atmosphere. $BBr_3$ was added. The mixture was warmed to room temperature and $CH_3OH$ was added. The solvent was removed in vacuo to give 1,6-bis-[2-(3,4-dihydroxybenzyl)-pyrrolidin-1-yl]hexane.

### STEP 3A

1,6-Bis{2-(3,4-dihydroxybenzyl)pyrrolidin-1-yl}hexane dihydrobromide

A solution of the methoxy compound obtained from STEP 2 (2.2 g, 4.4 mmol) was heated in 48% hydrobromic acid (11 ml) at reflux temperature for 3 h (followed by TLC on silica gel using basic mixture as developing solvent). The solution was cooled to room temperature and the aqueous phase was decanted from the oil which separated from solution. Toluene was added to this oil and then removed in vacuo. This addition removal using toluene was repeated several times to remove water and hydrogen bromide. The residual solid, 1,6-Bis{2-(3,4-dihydroxybenzyl)pyrrolidin-1-yl} hexane, a mixture of RR, SS and meso isomers was obtained as a foam. It should be noted that the specific procedure provided a dihydrobromide of the end product.

In a similar manner, using the appropriate ZXZ compound and following steps 1 through 3 above, the following compounds can be obtained:
(±)1,2-bis[2(3,4-dihydroxybenzyl)pyrrolidin-1-yl]-ethane;
(±)1,3-bis[2(3,4-dihydroxybenzyl)pyrrolidin-1-yl]-propane;
(±)1,4-bis[2(3,4-dihydroxybenzyl)pyrrolidin-1-yl]-butane;
(±)1,5-bis[2(3,4-dihydroxybenzyl)pyrrolidin-1-yl]-pentane;
(±)1,7-bis[2(3,4-dihydroxybenzyl)pyrrolidin-1-yl]-heptane;
(±)1,8-bis[2(3,4-dihydroxybenzyl)pyrrolidin-1-yl]-octane;
(±)1,9-bis[2(3,4-dihydroxybenzyl)pyrrolidin-1-yl]-nonane; and
(±)1,10-bis[2(3,4-dihydroxybenzyl)pyrrolidin-1-yl]-decane.

REACTION SCHEME II

DETAILED DESCRIPTION OF STEPS (a)-(h)

STEP (a)

In a 500 ml round bottom flask was placed 20 g (0.174 moles) of 1′ which is R-proline. To this was added 100 ml dry THF and 50 g (0.34 moles) of ethyl trifluoroacetate. The solution/flask was purged with argon and 30 g (0.261 moles) 1,1,3,3-tetramethylguanidine was added dropwise. The solution was allowed to stir until all the R-proline (1′) had dissolved (approximately 35 minutes). The solvent was removed in vacuo and the residue dissolved in $CH_2Cl_2$ (200 ml). The solution was washed with 6N HCl (aqueous, 2 x 100 ml). The organic layer was separated and dried with $Na_2SO_4$, filtered and the solvent removed in vacuo to give an oil which crystallized upon standing to give 1-trifluoroacetyl-R-proline.
Yield: 29 g, 79% mp, 48-51 °C.

STEP (b)

29 g of 1-trifluoroacetyl-R-proline (0.137 moles) was dissolved in $CH_2Cl_2$ (300 ml) in a 1 liter round bottom flask and cooled to 0 °C. To this solution was added 36.9 g (0.137 moles) diphenyl chlorophosphate followed by 15.27 g (0.151 moles) 4-methylmorpholine. After stirring at 0 °C for 10 minutes, the reaction mixture was allowed to warm to room temperature and stirred for an additional 10 minutes. The solution was then diluted with 600 ml dry diethyl ether. After filtration, the filtrate was washed with a saturated solution of $NaHCO_3$ (aq) and then the organic was layer separated, dried with $MgSO_4$, filtered and the solvent removed in vacuo to give a solid R-2-carboxy-1-trifluoroacetyl pyrrolidine anhydride with diphenylphosphoric acid which was hydroscopic and unstable to heat and moisture (90%, 54.8 g).

STEP (c)

To a 250 ml round bottom flask was added 4.51 g (0.186 moles) Mg° turnings and 100 ml dry THF. $I_2$ - (one crystal) was added, followed by 27 g (0.124 moles) 4-bromoveratrole dropwise. The reaction was heated under reflux after addition of the first few drops of 4-bromoveratrole until the iodine color disappeared. Following this, the remainder of 4-bromoverabrole was added dropwise. A condition of reflux was maintained for 2 hours. The solution was then cooled to room temperature and added to a solution of 54 g (0.123 moles) R-2-carboxy-1-trifluoroacetyl pyrrolidine anhydride with diphenylphosphoric acid in 250 ml dry THF (tetrahydrofuran) at -70° C. The rate of addition was monitored to keep the reaction temperature below -60° C. Once addition was complete the reaction mixture was warmed to room temperature and allowed to stir for 14 hours. It was then poured into a saturated solution of ammonium chloride (500 ml) and shaken. The organic layer was separated and dried with $MgSO_4$, filtered, and the solvent removed in vacuo to give an oil. This oil was subjected to flash chromatography in 1:1 hexane:ethyl acetate to give 20.4 g (50%) of formula (6′) R-2-(3,4-dimethoxybenzoyl)-1-trifluoroacetyl pyrrolidine, mp 121-123° C[α]-$_D^{25}$ = 65° c = 1.2($CHCl_3$).

STEP (d)

4.9 g (0.015 moles) R-2-(3,4-dimethoxybenzoyl)-1-trifluoroacetyl pyrrolidine was dissolved in 50 ml dry $CH_2Cl_2$ in a 500 ml round bottom flask. To the solution was added 20 g (0.172 moles) triethylsilane and 50 ml $BF_3 \cdot Et_2O$. The reaction mixture was stirred at room temperature for 3 days, after which time a saturated solution of potassium carbonate was added cautiously in a dropwise manner. When no more gas was evolved as a result of addition of $K_2CO_3$, 100 ml $CH_2Cl_2$ was added. The mixture was shaken. The triphasic mixture was filtered through a glass fritted funnel and the organic layer separated and dried with $MgSO_4$. Removal of the solvent in vacuo in yield R-2-(3,4-dimethoxybenzyl)-1-trifluoroacetyl pyrrolidine as an oil (3.2 g, 68%) which could be used without further purification.

STEP 5(e)

To a solution of 3.2 g (0.010 moles) R-2-(3,4-dimethoxybenzyl)-1-trifluoroacetyl pyrrolidine in 50 ml isopropyl alcohol was added 15 ml of 12.5 M HCl. The mixture was then heated under reflux until all starting material had disappeared, approximately 24 hours. The solvent was removed in vacuo to give an oil which could be recrystallized from isopropyl alcohol-ether to give needles of R-2-(3,4-dimethoxybenzyl)-pyrrolidine, (2.5 g, 97%), m.p. 165-167° C.

STEP (f)

To a 100 ml round bottom flask added 0.75 g (0.003 moles) R-2-(3,4-dimethoxybenzyl)pyrrolidine, 35 ml $CH_2Cl_2$, and 0.002 moles of $ClC(O)(CH_2)_4C(O)Cl$. The solution was cooled to 0° C and 0.911 g (0.009 moles) triethylamine was added. The solution immediately clouded and was warmed to room temperature. After 1 hour at room temperature, the reaction was poured into 1N HCl (35 ml) and shaken. The organic layer was separated, dried with $Na_2SO_4$, filtered, and the solvent removed in vacuo to give an oil R,R-1,6-bis-[2-(3,4-dimethoxybenzyl)pyrrolidin-1-yl]adipic acid diamide which was purified via column chromatography on silica with 5% methanol in methylene chloride.

STEP (g)

0.001 moles R,R-1,6-bis-[2-(3,4-dimethoxybenzyl)-pyrrolidinin-1-yl]adipic acid diamide in dry THF was added to a solution containing 0.5 ml (0.005 moles) 10 M $BH_3 \cdot DMS$ and the THF was heated under reflux

in an atmosphere of argon. Beating was maintained for 2 hours, after which time the mixture was cooled and 10 ml anhydrous methanol was added dropwise. After gas evolution had subsided, 10 ml of saturated HCl-MeOH was added dropwise and the mixture heated under reflux for 15 minutes. The reaction was cooled to room temperature and the solvent removed *in vacuo*, leaving an oily residue whose volume was further reduced via Kugelrohr distillation (100° C, 0.05 torr). Finally, the residue was dissolved in isopropyl alcohol and ether was added until the solution became cloudy. Crystallization usually occurred overnight forming the compound R,R-1,6-bis-[2-(3,4-dimethoxybenzyl)pyrrolidin-1-yl]-hexane.

## STEP (h)

To a solution of R,R-1,6-bis-[2-(3,4-di-methoxybenzyl)pyrrolidin-1-yl]hexane (0.001 moles) in dry $CH_2Cl_2$ at -70° C under inert atmosphere was added 4 ml (0.004 moles) of a 1M solution of $BBr_3$. The reaction mixture was warmed to room temperature, cooled to -70° C, and $CH_3OH$ (4 ml) was added. After warming to room temperature, the solvent was removed *in vacuo* and the residue passed down a filtration column and the product was eluted with $CH_3OH$. The fractions containing the bis-catechol were concentrated *in vacuo* to provide R,R-1,6-bis[2-(3,4-dihydroxybenzyl)pyrrolidin-1-yl]hexane $[\alpha]_D^{25}$ = 12.86.

By following steps (f), (g) and (h), using the appropriate $ClC(O)(CH_2)_pC(O)Cl$ compound in which p is equal to m-2, where m is as defined above, for example p = 2, 3, 5, 6, 7 or 8, in step (f) the appropriate optically pure R- or S-proline, one obtains the following compounds:
R,R-1,4-bis[2-(3,4-dihydroxybenzyl)pyrrolidin-1-yl]-butane;
S,S-1,4-bis[2-(3,4-dihydroxybenzyl)pyrrolidin-1-yl]-butane;
R,R-1,5-bis[2-(3,4-dihydroxybenzyl)pyrrolidin-1-yl]-pentane;
S,S-1,6-bis[2-(3,4-dihydroxybenzyl)pyrrolidin-1-yl]-hexane;
R,R-1,7-bis[2-(3,4-dihydroxybenzyl)pyrrolidin-1-yl]-heptane;
S,S-1,7-bis[2-(3,4-dihydroxybenzyl)pyrrolidin-1-yl]-heptane;
R,R-1,8-bis[2-(3,4-dihydroxybenzyl)pyrrolidin-1-yl]-octane; and
S,S-1,8-bis[2-(3,4-dihydroxybenzyl)pyrrolidin-1-yl]-octane.

## DATA

### A. Goldberg Renal and Femoral *In Vivo* Dog Model

The compounds were subsequently tested following intra-arterial administration in an *in situ* renal and femoral arterial preparation in the dog, as developed by L.I. Goldberg, to test for DA1 and DA2 dopamine activity, respectively. Details of the methodology are provided in the European J. Pharmacol., 89:137, 1983, as well as in Hypertension, 6:1-25, 1984. These compounds were found to be highly active when compared to dopamine and di-propyl dopamine, the respective DA1 and DA2 standards for this preparation.

## SCREENING SUMMARY OF COMPOUNDS TESTED IN THE GOLDBERG RENAL AND FEMORAL IN VIVO DOG MODEL

## SUMMARY

The test compound ($R_1$ and $R_2$ = H; X = $(CH_2)_m$; m = 6) was tested in the Goldberg renal and femoral dog assays for $DA_1$ and $DA_2$ activity. In contrast to our $DA_2$ standard agent, the activity of some of these compounds has been difficult to characterize in the Goldberg femoral assay. This entire series of compounds, however, displays highly potent and specific $DA_1$ activity.

The compound was injected directly into the renal or femoral artery for analysis of $DA_1$ or $DA_2$ activity, respectively. Since the compound was introduced into the $DA_1$ and $DA_2$ receptor beds in minimal doses,

specific renal and femoral vasodilation was observed in the absence of systemic effects. The compound was tested for relative activity in comparison to the standard $DA_1$ agonist, dopamine or the standard $DA_2$ agonist, DPDA. Specific $DA_1$ or $DA_2$ activity of the tested compound was verified by Sch 23390 or domperidone blockade.

General

Adult mongrel dogs of either sex (11-20 kg) were anesthetized with pentobarbital sodium (33 mg/kg, i.v.). the animals were intubated and ventilated with room air using a Harvard respirator.

Renal

The abdominal aorta was catheterized with a Millar micro-tip (size: 5F) transducer via the right femoral artery for pressure monitoring. The right femoral vein was cannulated with PE-160 tubing for iv saline infusion, as well as, supplemental pentobarbital administration. The left renal artery was isolated through a lateral abdominal incision. An electromagnetic flowprobe of the appropriate size (9-12 mm circ) was placed around the artery and connected to a Carolina Medical electromagnetic flowmeter. Surface ECG leads were placed subcutaneously on the chest and limbs to monitor heart rate. A 25 gauge 3/4" needle bent 90°, 4 mm from the tip, was placed in the renal artery proximal to the probe and connected to an infusion pump delivering saline at a constant rate of 1 ml/min. Following a saline and norepinephrine NE, 5.92 nmoles, challenge, both given in an injection volume of 0.2 ml, the animal was treated with a phenoxybenzamine, 0.5 mg/kg/min i.a., infusion for a duration of 10-20 minutes as necessary to achieve a complete blockade of the NE challenge. Blood pressure was maintained by supplemental saline (10-20 ml/kg, iv). Bradykinin, a standard for nonspecific vasodilation, was given at 0.62 nmole doses, in 0.2 ml saline, i.a., prior to generating a dose response curve for dopamine in which 4-fold increase doses, ranging from 3 to 48 nmoles, were injected i.a. in 0.2 ml of saline. Test compound was injected similarly in 4-fold increasing doses up to a maximum of 3000 nmoles. Compound was then repeated following Sch 23390 pretreatment (0.05 - 0.10 mg/kg, iv).

Femoral

The thoracic aorta was catheterized with a Millar micro-tip (size 5F) transducer via the right carotid artery for pressure monitoring. The right external jugular vein was cannulated with PE-160 tubing for supplemental pentobarbital administration. The left femoral artery was isolated and an electromagnetic flowprobe of the appropriate size (8-12 mm circ) was placed around the artery and connected to a Caroline Medical electromagnetic flowmenter. Surface ECG leads were placed subcutaneously on the chest and limbs to monitor heart rate. A 25 guage 3/4" needle bent 90°, 4 mm from the tip, was placed in the femoral artery proximal to the probe and connected to an infusion pump delivering saline at a constant rate of 1 ml/min. Following a saline and bradykinin challenge (0.02 or 0.04 nmoles, in 0.2 ml saline, i.a.), a dose response curve for DPDA was generated by injecting 4-fold increasing doses ranging from 3 to 190 nmoles in 0.2 ml of saline. Test compound was injected similarly in 4-fold increasing doses up to 3000 nmoles. Compound was then repeated following domperidone pretreatment (10-60 ug/kg, iv).

## DATA ANALYSIS

The optimal dose of compound was defined as that which produced a maximum increase in blood flow before causing a change in systemic blood pressure. Efficacy ratios were determined by division of the optimal response of the test compound by the optimal response of the standard agent. $ED_{50}$ values were derived by regression analysis from individually generated dose response curves. Potency ratios were the quotients calculated from the division of the standard agent $ED_{50}$ by the $ED_{50}$ of the test compound. This data together with the information on the percent blockade of optimal doses of the active compounds following antagonist administration is present in the following Table. If the inhibition of the test compound was similar to that of dopamine or DPDA, the compound was considered to be a $DA_1$ or $DA_2$ agonist,

respectively.

| RENAL $DA_1$ ASSAY | | | | | |
|---|---|---|---|---|---|
| Compound | N | $ED_{50}$ (nmoles) | Relative Potency to $DA_1$ Standard | Relative Efficacy to $DA_1$ Standard | Percent Block Following SCH 23390 |
| Standard : | | | | | |
| Dopamine | 12 | | 1.00 | 1.00 | 100% |
| Test Compound (R,R) | 3 | 0.63 | 11.71 | 1.05 | 100% |
| R = response was reversed following Schering 23390 blockage. | | | | | |

| RENAL $DA_2$ ASSAY | | | | | | |
|---|---|---|---|---|---|---|
| Compound | Entantiomer | N | $ED_{50}$ (nmoles) | Relative Potency to $DA_2$ Standard | Relative Efficacy to $DA_2$ Standard | Percent Block Following Domperidone |
| Standard : | | | | | | |
| DPDA | | 21 | 1.00 | 1.00 | 1.00 | 100% |
| test compound | R,R | 1 | 0.19 | 58.63 | 0.60 | 60% |
| test compound | R,R | 2 | slight local constriction | | | |
| (c) = local constriction prior to local dilation. | | | | | | |

B. Spontaneous Hypertensive Rat-Diuretic Activity

Additionally, these compounds were examined for diuretic activity for 6 hours following oral compound administration in the saline-loaded spontaneously hypertensive rat (SHR). Methods for this assay have been previously described (Rosenkranz, et al, Proc. West. Pharmacol. Soc. 28:87, 1985). The diuretic and natriuretic effects elicited by these compounds were characterized by an immediate onset of action. Over the course of the 6 hour study, these compounds were found to be as efficacious as the standard diuretic agent, hydrochlorothiazide.

Male spontaneously hypertensive SHR/NCr1BR rats weighing 320-430 gms were divided into four groups of seven animals. All animals were food- and water-deprived overnight. The following morning, each group of rats was hydrated with deionized water (20 ml/kg, po) forty-five minutes prior to the administration of vehicle or the test compound ($R_1$ and $R_2$ = H; X = $(CH_2)_n$, m = 6).

Following vehicle or drug, the rats were placed in individual metabolic units. Fifteen minutes post-dose the animals were saline-loaded (30 ml/kg, po). Urine was collected at 1, 3 and 6 hour intervals post-saline-load. Urine volumes were measured and sodium and potassium levels were determined by flame photometry.

A two-way analysis of variance with time, treatment and their interaction was run as a repeated measures analysis. A secondary model of one-way analysis of variance (by time) was run using specified contrasts. The p-values for the contrasts were adjusted using Fisher's LSD strategy at each time point.

One hour post oral administration of the test compound at 10 mg/kg, significant diuresis and natriuresis were observed. diuresis and natriuresis ($p<0.05$) were observed at the 30 mg/kg dose level at 1, 3 and 6 hours post-dose. Administration of 30 mg/kg, po, produced significant kaliuresis at the 1 and 3 hour post-dose time points. The lowest dose (3 mg/kg, po) elicited significant urine and sodium retention at 1 hour and 1 and 3 hours, respectively.

| THE EFFECTS ON URINE VOLUME, SODIUM AND POTASSIUM EXCRETION IN THE SPONTANEOUSLY HYPERTENSIVE RAT | | | | |
|---|---|---|---|---|
| | Urine Volume (ml) n = 7 $\overline{X}$ % S.D. | | | |
| Cumulative Time (hr) | Control Vehicle[b] | Test Compound | | |
| | | 3 mg/kg, po | 10 mg/kg, po | 30 mg/kg, po |
| 1 | 5.5 % 1.0 | 2.8 % 1.0* | 7.6 % 1.3* | 13.9 % 2.0* |
| 3 | 8.6 % 0.9 | 7.5 % 1.5 | 10.1 % 3.2 | 22.4 % 1.8* |
| 6 | 9.6 % 0.8 | 9.4 % 2.3 | 11.0 % 3.2 | 22.8 % 1.8* |
| | Urine $Na^+$ (mEq/sample) n = 7 $\overline{X}$ % S.D. | | | |
| Cumulative Time (hr) | Control Vehicle[b] | Test Compound | | |
| | | 3 mg/kg, po | 10 mg/kg, po | 30 mg/kg, po |
| 1 | 0.01 % 0.01 | 0.00 % 0.00* | 0.03 % 0.01* | 0.21 % 0.10* |
| 3 | 0.09 % 0.06 | 0.03 % 0.05* | 0.13 % 0.07 | 0.62 % 0.20* |
| 6 | 0.17 % 0.11 | 0.16 % 0.13 | 0.22 % 0.10 | 0.73 % 0.19* |
| | Urine $K^+$ (mEq/sample) n = 7 $\overline{X}$ % S.D. | | | |
| Cumulative Time (hr) | Control Vehicle[b] | Test Compound | | |
| | | 3 mg/kg, po | 10 mg/kg, po | 30 mg/kg, po |
| 1 | 0.04 % 0.01 | 0.02 % 0.01 | 0.04 % 0.01 | 0.08 % 0.04* |
| 3 | 0.11 % 0.03 | 0.08 % 0.03 | 0.11 % 0.05 | 0.20 % 0.09* |
| 6 | 0.17 % 0.06 | 0.17 % 0.05 | 0.18 % 0.07 | 0.25 % 0.09 |

*$p<0.05$ as compared to control (SAS statistical analysis program).
[b]2% ethanol and 0.5% tween in deionized water.

C. Spontaneous Hypertensive Rats - Antihypertensive Activity

The compounds of this invention were screened for oral antihypertensive activity in the conscious restrained rat. Adult male SHR were instrumented for blood pressure and heart rate measurements under light ether anesthesia. The animals were maintained on a plexiglass restraining board following surgery, and were allowed a 1 hour recovery period prior to the oral administration of the test compounds. These compounds were found to decrease blood pressure in the absence of a reflex tachycardia for up to 4 hours post-dosing.

Male spontaneously hypertensive rats, SHR/NVr1Br (320-410 g) were fasted overnight. The following morning the left femoral artery and vein of each rat was cannulated under light ether anesthesia. following surgery, the rats were placed on a rat restraining board and allowed to recover from the effects of the ether for 1 hour. ECG leads were placed on the animal's chest to monitor heart reate. Animals were divided into groups of 4 and dosed with the test compound ($R_1$ and $R_2$ = H; X = $(CH_2)_m$; m = 6).

Blood pressure and heart rate readings were taken at 5 minute intervals for the first 15 minutes and every 15 minutes thereafter for a duration of 4 hour post-compound administration. Data was evaluated by two-tailed paired t-tests.

The test compound ($R_1$ and $R_2$ = H; X = $(CH_2)_m$; m = 6) (3, 10 and 30 mg/kg, po) elicited a significant decrease in MBP. Peak decreases ranged from -17 to -22% while duration of action lasted between 15-210 minutes. HR was significantly increased following administration of the test compound 3 mg/kg, po, (6% FOR 90 minutes). Conversely, 10 mg/kg, po, decreased ($p<0.05$) HR for 1 hour post-compound administration (max. response of -14%). 30 mg/kg, po, did not elicit any biologically significant changes in HR (Figure 4).

D. Toxicity

Rats were administered compound I ($R_1$ and $R_2$ = H; X = $(CH_2)m$; m = 6; R,R isomer) in doses of 30 and 100 mg/kg once a day for 7 days. All rats survived for the duration of the treatment. No treatment related changes were noted in clinical condition, body weight, or food intake.

The instant invention is disclosed and described herein in what is considered to be the most practical, and preferred embodiments. It is recognized, however, that departures may be made therefrom which are within the scope of the invention, and that obvious modifications will occur to one skilled in the art upon reading this disclosure.

## Claims

1. A compound of formula (I) including mixtures of optically active compounds and optically pure R and S stereoisomers

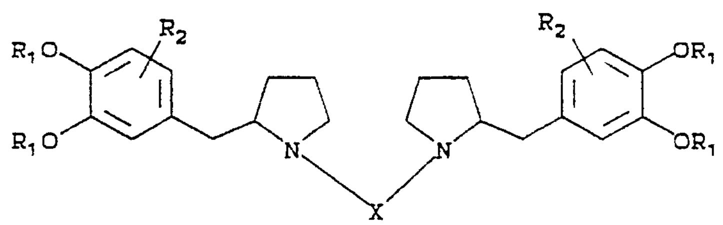

(I)

wherein:

$R_1$ is independently hydrogen, lower alkyl, $-C(O)R_3$, $-C(O)NR_3R_4$, wherein $R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;

$R_2$ is hydrogen or lower alkyl;

X is $-(CH_2)_m-$ or $-(CH_2)_nY(CH_2)_n-$, where m is an integer from 1 to 10, n is an integer of 1 to 3 and Y is oxygen or sulfur;

and a pharmaceutically acceptable salt or ester thereof.

2. The compound of Claim 1 wherein each $R_1$ and $R_2$ is hydrogen.

3. The compound of Claim 2 wherein X is $-(CH_2)_m-$and m is an integer from 4 to 8.

4. The optically pure R,R stereoisomer of the compound of Claim 3.

5. The compound of Claim 4, wherein m is 6, namely R,R-1,6-bis-[2-(3,4-dihydroxybenzyl)pyrrolidin-1-yl]hexane, or a pharmaceutically acceptable salt thereof.

6. A composition, comprising:

a pharmaceutically acceptable carrier; and

a compound of formula (I) including mixtures of optically active compounds and optically pure R and S stereoisomers:

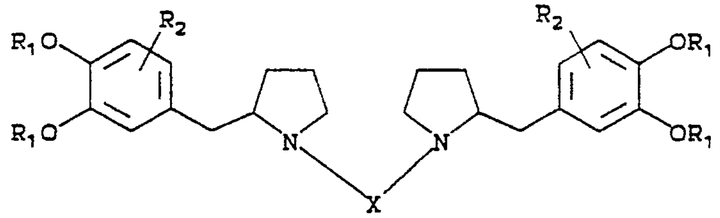

(I)

wherein:

$R_1$ is independently hydrogen, $-C(O)R_3$, $-C(O)NR_3R_4$, wherein $R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl

and halo;

$R_2$ is hydrogen or lower alkyl;

X is $-(CH_2)_m-$ or $-(CH_2)_nY(CH_2)_n-$, where m is an integer from 1 to 10, n is an integer of 1 to 3 and Y is oxygen or sulfur;

or a pharmaceutically acceptable salt or ester thereof.

7. The composition as claimed in Claim 6, wherein the composition comprises a pharmaceutically acceptable salt of the compound of formula (I) and $R_1$ and $R_2$ are each hydrogen.

8. The optically pure R,R stereoisomers of the composition as claimed in Claim 7.

9. A process for the preparation of compounds of formula I:

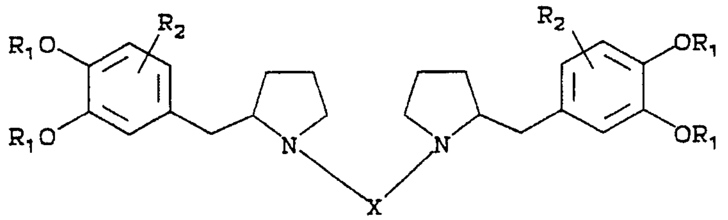

(I)

wherein:

$R_1$ is independently hydrogen, lower alkyl, $-C(O)R_3$, $-C(O)NR_3R_4$, wherein $R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;

$R_2$ is hydrogen or lower alkyl;

X is $-(CH_2)_m-$ or $-(CH_2)_nY(CH_2)_n-$, where m is an integer from 1 to 10, n is an integer of 1 to 3 and Y is oxygen or sulfur;

or a pharmaceutically acceptable salt or ester thereof, which comprises

a) reacting a compound of formula II

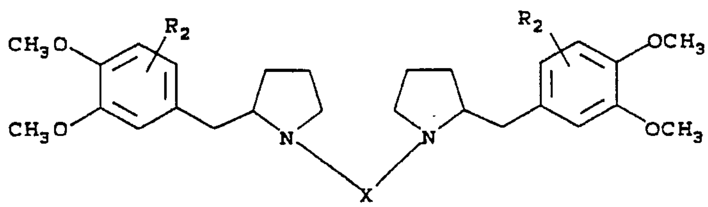

(II)

wherein $R_2$ and X are as defined above with a deprotecting agent; or

b) converting a compound of formula I to a pharmaceutically acceptable salt of formula I; or

c) converting a pharmaceutically acceptable salt of formula I to a free compound of formula I; or

d) converting a pharmaceutically acceptable salt of formula I to another pharmaceutically acceptable salt of formula I; or

e) converting a compound of formula I to an ester of formula I.

10. A compound of the formula:

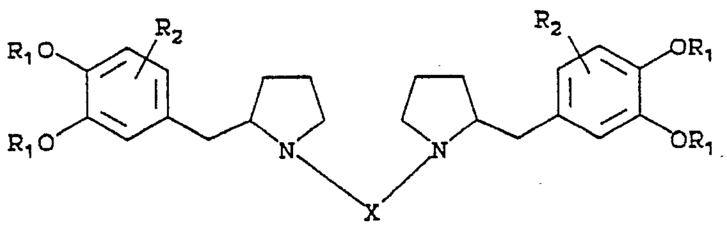

(I)

wherein:

$R_1$ is lower alkyl,

$-C(O)R_3$, $-C(O)NR_3R_4$, wherein
$R_3$ and $R_4$ are independently lower alkyl, optionally substituted phenyl or phenyl lower alkyl wherein the substitution may be mono- or disubstitution with a substituent selected from the group consisting of lower alkyl, lower alkoxy, hydroxy, trifluoromethyl and halo;
$R_2$ is hydrogen or lower alkyl;
X is $-(CH_2)_m-$ or $-(CH_2)_nY(CH_2)_n-$, where m is an integer from 1 to 10, n is an integer of 1 to 3 and Y is oxygen or sulfur;
or a pharmaceutical.ly acceptable salt or ester thereof.

11. The use of a compound according to any one of claims 1 to 5 in the manufacture of a medicament for treating hypertension.

12. A compound of formula:

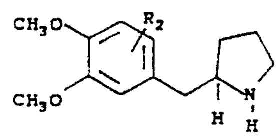

wherein:
$R_2$ is hydrogen or lower alkyl;
and a pharmaceutically acceptable salt or ester thereof.

European Patent Office

# EUROPEAN SEARCH REPORT

Application number

EP 90111675.6

| DOCUMENTS CONSIDERED TO BE RELEVANT | | | |
|---|---|---|---|
| Category | Citation of document with indication where appropriate, of relevant passages | Relevant to claim | CLASSIFICATION OF THE APPLICATION (Int Cl.⁵) |
| X | DE - A1 - 2 733 753 (BYK) * Example 22 * -- | 1,11, 12 | C 07 D 207/08<br>A 61 K 31/40 |
| X | DE - A1 - 2 733 817 (BYK) * Example 22 * -- | 1,11, 12 | |
| X | CHEMICAL ABSTRACTS, vol. 101, no. 21, November 19, 1984, Columbus, Ohio, USA NORDLANDER, J. ERIC et al. "Friedel-Crafts acylation with N-(trifluoroacetyl) -alpha-amino acid chlorides. Application to the preparation of beta-arylalkylamines and 3-substituted 1,2,3,4-tetrahy- droisoquinolines" page 744, column 2, abstract- -no. 191 656e & J. Org. Chem. 1984, 49(22), 4107-11 (Eng) -- | 12 | TECHNICAL FIELDS SEARCHED (Int Cl.⁵)<br>C 07 D 207/00 |
| A | CHEMICAL ABSTRACTS, vol. 103, no. 19, November 11, 1985, Columbus, Ohio, USA EHRING, ERLING et al. "Preparation of carbon-11- -labeled raclopride, a new potent dopamine receptor antagonist: preliminary PET studies of cerebral dopamine receptors in the monkey" page 692, column 1, abstract- -no. 160 330f & Int. J. Appl. Radiat. Isot. 1985, 36(4), 269-73 (Eng) -- | 1,6,11 | |
| A | CHEMICAL ABSTRACTS, vol. 105, | 1,6,11 | |

The present search report has been drawn up for all claims

| Place of search | Date of completion of the search | Examiner |
|---|---|---|
| VIENNA | 03-09-1990 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number

-2-

EP 90111675.6

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| | no. 7, August 18, 1986, Columbus, Ohio, USA BRUBAKER, ABRAM N. et al. "Synthesis and pharmacological evaluation of some 6-substituted 7-methyl-1,4-dioxa-7-azaspiro(4.5)decanes as potential dopamine agonists" page 610, column 1, abstract-no. 60 556g & J. Med. Chem. 1986, 29(8), 1528-31 (Eng) -- | | |
| A | CHEMICAL ABSTRACTS, vol. 97, no. 19, November 8, 1982, Columbus, Ohio, USA FLORVALL, LENNART et al. "Potential neuroleptic agents. 2,6-Dialkoxybenzamide derivatives with potent dopamine receptor blocking activities" page 25, column 2, abstract-no. 155 943m & J. Med. Chem. 1982, 25(11), 1280-6 (Eng) ---- | 1,6,11 | |

TECHNICAL FIELDS SEARCHED (Int Cl 5)

The present search report has been drawn up for all claims

| Place of search | Date of completion of the search | Examiner |
|---|---|---|
| VIENNA | 03-09-1990 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document